(19) Europäisches Patentamt
European Patent Office
Office européen des brevets

(11) **EP 4 251 050 B1**

(12) **EUROPEAN PATENT SPECIFICATION**

(45) Date of publication and mention
of the grant of the patent:
**07.01.2026 Bulletin 2026/02**

(21) Application number: **21827302.7**

(22) Date of filing: **19.11.2021**

(51) International Patent Classification (IPC):
*A61B 5/30* (2021.01)    *A61B 5/00* (2006.01)
*G16H 20/30* (2018.01)    *G16H 40/63* (2018.01)
*H03K 5/1252* (2006.01)    *A61B 5/31* (2021.01)
*A61B 5/308* (2021.01)    *A61B 5/367* (2021.01)

(52) Cooperative Patent Classification (CPC):
**A61B 5/30; A61B 5/7203; A61B 5/7257;
A61B 5/7278; G16H 20/30; G16H 40/63;
H03K 5/1252;** A61B 5/308; A61B 5/31; A61B 5/367

(86) International application number:
**PCT/US2021/060023**

(87) International publication number:
**WO 2022/115317 (02.06.2022 Gazette 2022/22)**

(54) **NOISE FILTERING FOR ELECTROPHYSIOLOGICAL SIGNALS**

RAUSCHFILTERUNG FÜR ELEKTROPHYSIOLOGISCHE SIGNALE

FILTRAGE DE BRUIT POUR SIGNAUX ÉLECTROPHYSIOLOGIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.11.2020 US 202063118213 P**
**09.06.2021 US 202117342718**

(43) Date of publication of application:
**04.10.2023 Bulletin 2023/40**

(73) Proprietor: **CardioInsight Technologies, Inc.**
**Independence, OH 44131 (US)**

(72) Inventors:
• **ZENG, Qingguo**
**Independence, Ohio 44131 (US)**
• **ADAIR, Jeffrey B.**
**Independence, Ohio 44131 (US)**
• **GEORGE, Brian P.**
**Independence, Ohio 44131 (US)**
• **LOU, Qing**
**Independence, Ohio 44131 (US)**
• **LASKE, Timothy G.**
**Independence, Ohio 44131 (US)**

(74) Representative: **Zimmermann & Partner**
**Patentanwälte mbB**
**Postfach 330 920**
**80069 München (DE)**

(56) References cited:
US-A1- 2014 278 171    US-A1- 2017 071 675
US-A1- 2019 183 425    US-A1- 2019 261 927

• JOHANN GLASER ET AL: "FACET - a "Flexible
Artifact Correction and Evaluation Toolbox" for
concurrently recorded E", BMC
NEUROSCIENCE, BIOMED CENTRAL, LONDON,
GB, vol. 14, no. 1, 9 November 2013 (2013-11-09),
pages 138, XP021167587, ISSN: 1471-2202, DOI:
10.1186/1471-2202-14-138

## Description

## TECHNICAL FIELD

This disclosure relates to noise filtering of electrophysiological signals.

## BACKGROUND

Electrophysiological signals are sensed in a variety of applications, including electroencephalography, electrocardiography, electromyography, electrooculography and the like. Electrophysiological signals are often contaminated by noise, such as power line noise. Contaminants in electrophysiological signals can reduce signal quality, which in some applications, can impact subsequent signal processing and diagnosis.

Documents US 2019/183425 A1, US 2014/278171 A1, and Glaser et al., BMC Neuroscience vol. 14, no. 1, page 138 (2013) disclose approaches for noise filtering in electrophysiological signals by subtracting noise signals.

## SUMMARY

This disclosure relates to noise filtering of electrophysiological signals.

According to the invention, one or more non-transitory computer-readable media includes data and machine readable instructions that can be executed by a processor. The data includes electroanatomical data characterizing an electrophysiological signal measured from a patient. The machine readable instructions include a signal segment extractor programmed to extract a signal segment of interest from the electrophysiological signal, a signal segment noise calculator programmed to evaluate the extracted signal segment of interest to estimate a noise in the signal segment of interest, and a signal segment filter programmed to determine a surrogate noise estimate for at least one remaining signal segment of the electrophysiological signal and filter the at least one remaining signal segment to remove noise therein based on the surrogate noise estimate. The signal segment extractor is further programmed to apply a moving window function to sample a portion of the electrophysiological signal and evaluate a signal morphology of the sampled portion of the electrophysiological to determine whether the portion of the electrophysiological signal is to be identified as the signal segment of interest.

## BRIEF DESCRIPTION OF THE DRAWINGS

FIG. 1 depicts an example of a noise filtering system for electrophysiological signals.
FIG. 2 depicts an example of a signal processing system for electrophysiological signals.
FIG. 3 depicts an example of electrophysiological signals and frequency-spectrum plots.
FIGS. 4A-4E depict examples of electrophysiological signal and noise filtering plots.
FIG. 5 depicts an example of electrophysiological signal plots.
FIG. 6 depicts an example of an electrophysiological monitoring system that can implement electrophysiological noise filtering.
FIG. 7 illustrates an example of an electrophysiological map.
FIG. 8 illustrates another example of an electrophysiological map.
FIG. 9 illustrates an example of a method for filtering noise from an electrophysiological signal.

## DETAILED DESCRIPTION

This disclosure relates to signal filtering that can be applied to reduce noise from sensed electrophysiological signals. An electrophysiological noise filter can be configured to remove white noise (e.g., Gaussian noise), fixed frequency noise (e.g., power line noise), harmonic noise, and/or transient noise (e.g., spike noise), for example. The term "noise" as used herein can refer to an unwanted signal or artifact in an electrophysiological signal.

In some examples, non-claimed systems and methods are provided for removing a noise in electrophysiological signals, such as can be stored in memory as electrical data. The electrical data can correspond to a digital representation of one or more electrophysiological signals that have been measured from an individual's body by one or more electrodes or other sensors (e.g., contact or non-contact sensors), such as unipolar or bipolar electrograms, or other electrophysiological signals. By way of example, a signal segment extractor (e.g., executable program code) is employed to extract a signal segment of interest from an electrophysiological signal and provide the extracted signal segment to a signal segment noise calculator. In some examples, the identified signal segment of interest can correspond to a portion of the electrophysiological signal that does not contain a noise distorting feature, such as a transient feature (e.g., a spike) and/or a QRS feature.

The signal segment noise calculator (e.g., executable program code) can be configured to evaluate the extracted signal segment of interest and estimate a noise in the signal segment of interest. A signal segment filter (e.g., executable program code) can be configured to remove noise from at least one remaining signal segment of the electrophysiological signal based on the estimated noise in the signal segment of interest. For example, the signal segment filter is configured to compute a surrogate estimate for the remaining signal segments. As such, the noise identified in the signal segment of interest can be used to provide the surrogate noise estimate for each remaining signal segments of interest, and the signal segment filter can be configured to remove the noise in each remaining signal segment based on the surrogate noise estimate. The signal segment filter can be configured to remove the noise in the remaining signal segments based on the surrogate noise estimate and the signal segment of interest based on the estimated noise to remove the noise (e.g., power line noise) from the electrophysiological signal. By employing a portion of the electrophysiological signal that does not include noise distorting features for noise estimation can improve an accuracy of estimating noise in the electrophysiological signal and consequently a diagnosis accuracy.

By way of example, the systems and methods disclosed herein are used as part of a diagnostic and/or treatment workflow to facilitate the identification and treatment of arrhythmias (e.g., cardiac resynchronization therapy (CRT), premature ventricular contraction (PVC), ventricular tachycardia (VT), and premature atrial contractions (PAC)) based on electrical activity acquired for the patient. In some examples, the patient electrical activity includes non-invasive body surface measurements of body surface electrical activity. Additionally or alternatively, the patient electrical activity can include invasive measurements of heart electrical activity, including epicardial measurements and/or endocardial measurements. While many examples herein are described in the context of cardiac electrical signals, it is to be understood that the approaches disclosed herein are equally applicable to other electrophysiological signals, such as electroencephalography, electromyography, electrooculography, and the like.

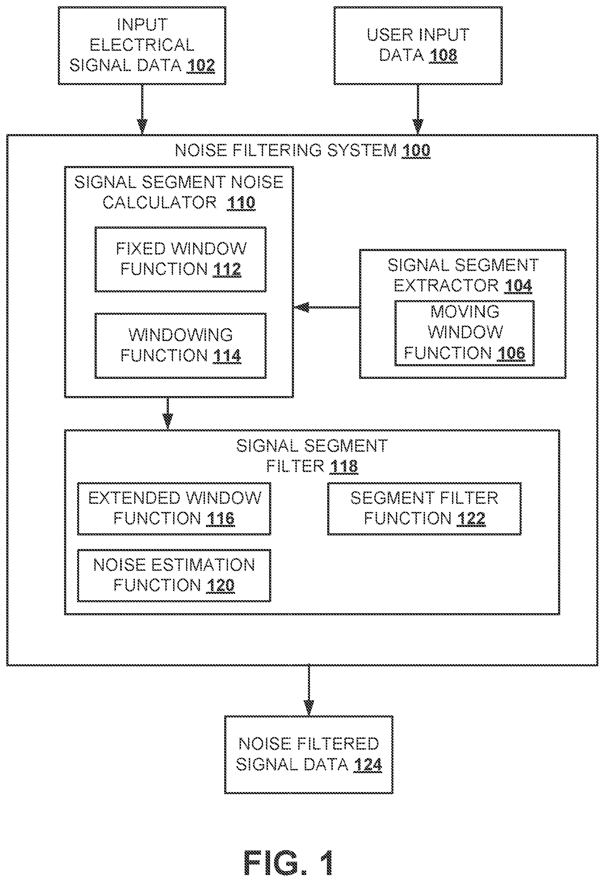

FIG. 1 depicts an example of a noise filtering system 100 that can be configured to filter input electrical signal data 102 to remove noise therein. The noise filtering system 100 can be implemented as hardware (e.g., circuit and/or devices), software (e.g., a non-transitory medium having machine-readable instructions), or a combination of hardware and software. The input electrical data 102 can be stored in memory and correspond to one or more electrophysiological signals, such as physiological signals obtained by one or more sensors. The sensors can be applied to measure the electrical activity non-invasively, such as may be positioned over a patient's body surface such as the patient's head (e.g., for electroencephalography), a patient's thorax (e.g., for electrocardiography), or other noninvasive locations. Examples of sensors that may be utilized to acquire the body surface electrical activity are disclosed in U.S. Pat. No. 9,549,683 and International application No. PCT/US20091063803. In other examples, the input electrical signal data 102 is acquired invasively, such as by one or more electrodes positioned within a patient's body (e.g., on a lead or a basket catheter during an EP study or the like). In yet other examples, the input electrical signal data 102 includes both non-invasively acquired electrical signals and invasively acquired electrical signals. In some examples, the one or more electrophysiological signals are acquired in real-time, such as during a procedure.

The noise filtering system 100 can be employed to remove noise from the one or more electrophysiological signals prior to low-pass filtering and transient feature identification and/or removal, such as spikes (e.g., pacing spikes). Thus, in some examples, the noise filtering system 100 is employed to provide initial line filtering prior to implementing other types of filtering on the one or more electrophysiological signals. In some examples, the noise filtering system 100 can be employed to remove unwanted signals in other signals generated by signal sources, such as therapeutic devices or navigation systems.

The noise filtering system 100 can be configured to evaluate one or more signal segments before and/or after a signal segment of interest of an electrophysiological signal of the one or more electrophysiological signals to remove noise from these signal segments based on estimated noise for the signal segment of interest. As used herein, the term "signal segment of interest" can refer to a portion of an electrophysiological signal in a frequency-domain that has discrete frequency components that are not within a respective frequency bin (e.g., frequency range). The signal segment of interest may be selected by a user (e.g., in response a user input) or automatically.

The respective frequency bin can be user-defined based on a noise filtering application in which the noise filtering system 100 is to be used. In some examples, if the noise filtering system 100 is employed as part of an electrophysiological monitoring system, the given frequency bin corresponds to a frequency range that does not include frequencies of a QRS signal component of the electrophysiological signal. Thus, the respective frequency bin can correspond to a frequency range from about 0 Hertz (Hz) to about 20 Hz, in some examples. As such, in some examples, the noise filtering system 100 is configured to filter line noise, such as power line noise having a frequency of about 50 Hz (e.g., such as in Europe, China, India, and the like) or about 60 Hz (e.g., such as in the United States of America).

In some examples, the noise filtering system 100 can include a machine learning algorithm configured to refine a filtering of the noise filtering system 100 based on historical noise filtering data (e.g., previous noise filtering settings). Any of a variety of techniques can be utilized for the machine learning algorithm, including support vector machines, regression models, self-organized maps, fuzzy logic systems, data fusion processes, rule-based systems, or artificial neural networks. In other examples, a different machine learning algorithm can be used. The machine learning algorithm can be trained based on the historical noise filtering data to adjust the noise filtering settings of the noise filtering system 100. The noise filtering setting can include signal morphology characteristics, such as used herein for signal segment extraction. In other examples, other settings of the noise filtering system 100 can be adjusted by the machine learning algorithm (e.g., padding function, transform function, window size, etc.).

By way of example, the noise filtering system 100 includes a signal segment extractor 104 to extract the signal segment of interest from the electrophysiological signal. For example, the signal segment extractor 104 includes program code configured to evaluate portions of the electrophysiological signal based on a moving window function 106. The moving window function 106 can have a defined window size representing a number of samples (e.g., sampling frequency) and a duration. The moving window function 112 can include a Hann window, a Hamming window, a Blackman window, Nuttall window, Blackman-Nuttall window, Blackman-Harris window, or another window-type function for signal sampling. The moving window function 106 can be configured to slide with respect to the electrophysiological signal to sample at least one signal portion of the electrophysiological signal. The signal segment extractor 104 can be configured to evaluate each sampled portion to determine whether a respective signal portion of the electrophysiological signal is to be identified or flagged as the signal segment of interest.

For example, the signal segment extractor 104 is configured to evaluate signal characteristics (e.g., a morphology) of each signal portion to identify the signal segment of interest. In some examples, the signal segment extractor 104 is configured to compare an amplitude and/or slope of each signal portion relative to a signal threshold (e.g., an amplitude and/or a slope threshold) to identify the signal segment of interest. The respective signal portion can be identified or flagged as the signal segment of interest based on the comparison. By way of example, if the amplitude of the respective signal portion is less than or equal to the amplitude threshold, the respective signal portion is identified or flagged as the signal segment of interest. In additional or alternative examples, if the slope of the respective signal portion is greater than or equal to the slope threshold, the respective signal portion can be identified or flagged as the signal segment of interest. Thus, in some examples, the signal segment extractor 104 is configured to evaluate the electrophysiological signal to identify signal segments that do not include noise distorting features (e.g., such as transient features (e.g., spikes) and/or QRS signal components).

In some examples, the signal segment extractor 104 is configured to extract the signal segment of interest for noise estimation based on user input data 108. The user input data 108 can identify a signal segment processing interval (e.g., a period of time with respect to the electrophysiological signal) for the extraction of the signal segment of interest. For example, an interval of the electrophysiological signal is rendered with respect to time on a display by a graphical user interface (GUI). The GUI can generate the electrophysiological signal with sliding scales that a user can interact with (e.g., through a user input device that provides user input data 108) to select the signal segment of interest and thus identify the segment processing interval for extraction by the signal segment extractor 104.

In some examples, electrical signals can be applied to the patient's body having different frequencies. For example, the electrical signals can be generated by a pacing device (e.g., a pacemaker). The input electrical signal data 102 can include data characterizing the applied electrical signals. The signal segment extractor 104 can be configured to evaluate the applied electrical signals to determine a common noise in the applied electrical signals. The signal segment extractor 104 can be configured to extract the signal segment of interest based on the determined common noise in the applied electrical signals.

The signal segment extractor 104 can be configured to provide the signal segment of interest to a signal segment noise calculator 110 for noise estimation. In some examples, the signal segment extractor 104 is configured to provide signal timing information identifying a start time and a stop time for the signal segment processing interval. The signal segment noise calculator 110 can be configured to employ the signal timing information to retrieve the signal segment of interest that is being stored in the memory. The signal segment noise calculator 110 can be configured to employ a fixed window function 112 to sample the signal segment of interest.

The signal segment noise calculator 110 can be configured to apply a transform function 112 to transform the signal segment of interest into a frequency domain representation. For example, the transform function 112 can be configured to apply discrete Fourier transform (DFT) to transform the signal segment of interest into a frequency domain representation. The signal segment noise calculator 110 can be configured to identify signals present in the signal segment of interest and represent the signals in the frequency domain as discrete frequency domain representations. The signal segment noise calculator 110 can be configured to compute a set of DFT coefficients for each signal present in the signal segment of interest. The signal segment noise calculator 110 can be configured to compute a respective DFT coefficient for each signal in the signal segment of interest to convert the signal segment of interest to corresponding frequency domain data. The corresponding frequency domain data can have discrete frequency bins for the signals in the signal segment of interest.

Each DFT coefficient provided by the signal segment noise calculator 110 can specify an amplitude and a phase of a discrete frequency domain representation, which collectively can define the signal segment of interest in the time-domain. The signal segment noise calculator 110 can be configured to represent the signal segment of interest in terms of complex exponentials according to a DFT series synthesis equation:

$$s[n] = \sum_{k=0}^{N-1} S[k]e^{j\frac{2\pi k}{N}n} \quad (1),$$

wherein $S[k]$ are DFT coefficients, $N$ is a period of a discrete-time signal $s[n]$, and $k$ is a number of exponentials.

The signal segment noise calculator 110 can be configured to determine the set of DFT coefficients according to a DFT analysis equation:

$$S[k] = \frac{1}{N} \sum_{\langle N \rangle} s[n]e^{-j\frac{2\pi k}{N}n} \quad (2),$$

wherein $\langle N \rangle$ indicates a sum over any N consecutive integers.

The signal segment noise calculator 110 can be configured to identify a DFT coefficient from the set of DFT coefficients for a noise signal representative of the noise that can be imposing on the signal segment of interest. For example, the signal segment noise calculator 110 is configured to employ the set of DFT coefficients to convert the signal segment of interest to a corresponding frequency domain representation. The frequency domain representation can correspond to a frequency-domain spectrum representing a power of frequency content that is present in the signals of the signal segment of interest. The signal segment noise calculator 110 can be configured to evaluate the frequency-domain spectrum to determine a frequency of the noise signal.

For example, the signal segment noise calculator 110 is configured to compare each frequency bin in the frequency spectrum to a frequency bin threshold. In some examples, the signal segment noise calculator 110 is configured to set the frequency bin threshold based on frequency bin criteria. The frequency bin criteria can specify a frequency of the noise signal (e.g., corresponding to a frequency of power line noise). In some examples, the frequency bin criteria is provided as or part of the user input data 108. The signal segment noise calculator 110 can be configured to identify a frequency of interest corresponding to the frequency of the noise signal in response to determining that the frequency of interest is equal to the frequency bin threshold.

The signal segment noise calculator 110 can be configured to select the DFT coefficient of the set of DFT coefficients for the noise signal based on the identified frequency of interest to estimate the noise in the signal segment of interest. As such, the signal segment noise calculator 110 can be configured to estimate the DFT coefficients for the noise signal and thereby estimate the phase and amplitude of the noise signal present in the signal segment of interest based on the identified frequency of interest. Accordingly, the signal segment noise calculator 110 can be configured to estimate the noise in the signal segment of interest, which can be stored in memory as signal segment noise data.

In some examples, the signal segment noise calculator 110 can be configured to apply a padding function 114 to the estimated noise corresponding to pad the estimated noise to change a frequency resolution of the estimated noise. Because an extended window function 116 for sampling the at least one remaining signal segment of the electrophysiological signal can have a different resolution than the estimated noise, the signal segment noise calculator 110 can be configured to pad the estimated noise to provide a padded version of the estimated noise. By way of example, the padding function 114 is configured to scale the DFT coefficients by a given window scale value based on window characteristics of the extended window function 116 to provide scaled DFT coefficients corresponding to changing the frequency resolution of the estimated noise.

The signal segment noise calculator 110 can be configured to provide the scaled estimated noise (corresponding to the scaled DFT coefficients) to a signal segment filter 118. The signal segment filter 118 can be configured to apply the extended window function 116 to select the at least one remaining signal segment of the electrophysiological signal for noise filtering. The signal segment filter 118 can employ a noise estimation function 120 that is configured to provide a surrogate noise estimate for the at least one remaining signal segment based on the scaled estimated noise. For example, the noise estimation function 120 is configured to interpolate the scaled estimated noise in the signal segment of interest to the remaining at least one signal segment of the electrophysiological signal to extrapolate noise in the remaining at least one signal segment. In some examples, the noise estimation function 120 is configured to extend and thus interpolate the phase and magnitude of the scaled estimated noise to the remaining at least one signal segment of electrophysiological signal to extrapolate the noise in the remaining at least one signal segment of electrophysiological signal. The surrogate noise estimate can be representative of the interpolated phase and magnitude of the scaled estimated noise for the remaining at least one signal segment of interest.

In some examples, the noise estimation function 120 is configured to estimate noise forward and backward with respect to the signal segment of interest to provide the surrogate noise estimate for the remaining signal segments based on the scaled estimated noise. For example, if the signal segment of interest is located temporally between a first remaining signal segment and a second remaining signal segment, the noise estimation function 120 is configured to predict backwards to provide the surrogate noise estimate for the first remaining signal segment and forwards to provide the surrogate noise estimate for the second remaining signal segment based on the scaled estimated noise with respect to the signal segment of interest. Accordingly, the noise estimation function 120 can provide the surrogate noise estimate for each remaining signal segment based on the estimated noise for the signal segment of interest.

In some examples, the signal segment filter 118 includes a segment filter function 122 configured to remove noise in each remaining signal segment based on the surrogate noise estimation provided by the noise estimation function 120. The segment filter function 122 can be configured to subtract the surrogate noise estimate for each remaining signal segment of interest from the respective signal segment to filter (e.g., remove) noise therein and provide filtered remaining signal segments. For example, the segment filter function 122 is configured to subtract noise in the signal segment of interest from the estimated noise to filter the signal segment of interest for the noise to provide a filtered signal segment of interest. The segment filter function 122 can be configured to combine (e.g., stitch) the filtered remaining signal segments and the filtered signal segment to provide a noise-filtered representation of the electrophysiological signal. The noise-filtered electrophysiological signal can be provided (and stored in memory) as noise filtered signal data 124.

The noise filtered signal data 124 can be used in further signal processing (e.g., signal conditioning), such as low-pass filtering or other filtering to provide filtered electrophysiological signal data. Additional signal processing techniques can also be utilized to provide such filtered electrophysiological signal data. As an example, the signal processing techniques can include electrogram reconstruction onto an epicardial or other envelope, such as by solving an inverse solution based on geometry data and electrical data measured over a body surface.

In some examples, the noise filtering system 100 is configured to filter noise in each channel employed to measure (e.g., capture) electrical activity from a human based on the estimated noise in the signal segment of interest. For example, a measurement system can be employed to capture electrical activity from a human's body via sensors (e.g., electrodes). Each sensor thus can define a respective channel. The measurement system can be configured to provide the captured electrical activity from the human's body for each channel as part of the input electrical signal data 102. A given electrophysiological signal provided by a respective channel can be selected and analyzed as described herein by the noise filtering system 100 to estimate a noise in a signal segment of interest of the given electrophysiological signal. In some examples, electrophysiological signals for any number of the channels can be rendered with respect to time on the display by the GUI with graphical elements that the user can interact with to select the given electrophysiological signal for noise estimation. In other examples, the signal segment extractor 104 is configured to select the given electrophysiological signal.

The noise filtering system 100 can be configured to estimate a noise in each channel based on the estimated noise in the signal segment of interest for the respective channel. For example, the noise estimation function 120 is configured to interpolate the estimated noise in the signal segment of interest of the given electrophysiological signal to each electrophysiological signal from respective remaining channels, and to extrapolate the noise in each electrophysiological signal as described herein. The segment filter function 122 can in turn remove the noise in each electrophysiological signal from respective remaining channels based on the estimated noise by the noise estimation function 120 to provide noise filtered electrophysiological signals. The noise filtered electrophysiological signals can be provided as the noise filtered signal data 124, in some examples.

In some examples, the sensors can be arranged on a body surface of the human and grouped into two or more proper subset of sensors for each of a plurality of spatial zones. Each spatial zone may include a subset of sensors. An electrophysiological signal from a given sensor in each spatial zone can be selected and evaluated by a respective noise filtering system, such as the noise filtering system 100, to estimate a noise in a signal segment of interest of the electrophysiological signal. Each respective noise filtering system can employ the estimated noise to filter the electrophysiological signal from the given sensor and electrophysiological signals from remaining sensors in each respective spatial zone to provide noise filtered electrophysiological signals. Thus, in some examples, for each spatial zone, a respective noise filtering system, such as the noise filtering system 100, can be employed to provide noise estimation and filtering in a same or similar manner as described herein.

In some examples, an analysis system as described herein can be configured to generate a first graphical map of electroanatomic activity based on electrophysiological signals provided by sensors of one or more first spatial zones of the plurality of spatial zones. The analysis system can be configured to generate a second graphical map of electroanatomic activity based on electrophysiological signals provided by sensors of one or more second spatial zones of the plurality of spatial zones. The analysis system can be configured to evaluate the first and second graphical maps of the electroanatomic activity to determine whether the noise has been sufficiently filtered for a respective one or more spatial zones of the plurality of spatial zones. For example, if a difference between the first and second graphical maps is a greater than a difference threshold, the analysis system can be configured to output on the GUI an indication that one of the first or the one or more second spatial zones is contaminated with noise. In some examples, the analysis system can be configured to cause the noise filtering system 100 to extract a different signal segment than the signal segment of interest for noise estimation.

Accordingly, the noise filtering system 100 can be employed to improve a signal quality of electrophysiological signals at an initial pre-processing stage which can reduce signal morphologies that can lead to a wrong diagnosis of arrhythmias. For example, electrograms are often contaminated by power line noise. The power line noise can overlap with frequencies of a QRS component of the electrogram, which can distort the power line noise. Employing the complete electrogram for noise estimation can lead to wrong line filtering results (e.g., incorrect nose estimations) as the noise can be distorted by the QRS portion of the electrogram. Moreover, transient events, such as pacing spikes that are captured together with electrograms and can also distort power line noise estimations. By employing the noise filtering system 100 to provide a power line noise estimation for the electrogram based on a portion of an electrogram that does not include the QRS signal component and/or the transient events allows for accurate estimation of power line noise. Accordingly, the noise filtering system 100 can accurately estimate the power line noise within the electrogram for improved power line noise filtering.

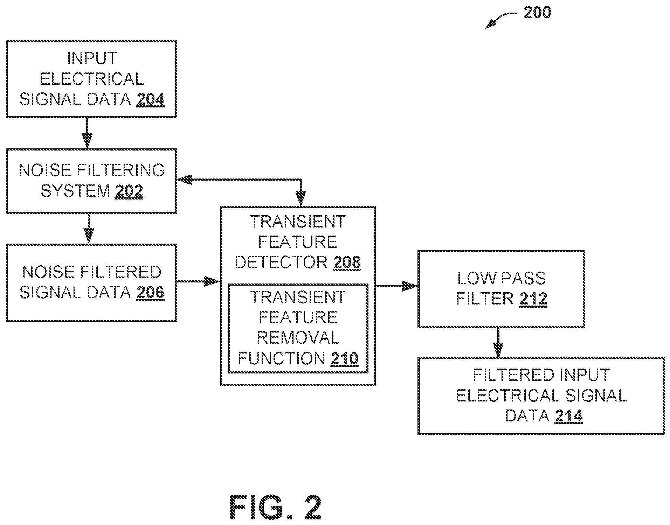

FIG. 2 depicts an example of a signal processing system 200 for electrophysiological signals. In some examples, the signal processing system 200 can be implemented on a processing device (e.g., a digital signal processor, a field-programmable gate array, computer, or other processing apparatus). The signal processing system 200 can be employed to process electrophysiological signals being provided by a respective channel from a measurement system employed to capture electrical activity from a human's body via one or more sensors. Thus, in examples wherein a plurality of sensors is employed to capture electrical activity from the human body, a respective signal processing system 200 can be employed to provide signal processing as described herein.

In the example of FIG. 2, the signal processing system 200 includes a noise filtering system 202 that receives input electrical signal data 204. In some examples, the noise filtering system 202 can correspond to the noise filtering system 100, as illustrated in FIG. 1. Therefore, reference can be made to the example of FIG. 1 in the following description of the example of FIG. 2. The noise filtering system 202 can be configured to filter the input electrical signal data 204 to provide noise filtered signal data 206 as described herein with respect to the noise filtering system 100. As such, in some examples, the input electrical signal data 204 and the noise filtered signal data 206 can correspond to the input electrical signal data 102 and the noise filtered signal data 124, as illustrated in FIG. 1. For example, the noise filtering system 202 is configured to receive electrophysiological signals and filter the electrophysiological signals based on estimated noise from a signal segment of interest of a given electrophysiological signal as described herein.

The signal processing system 200 includes a transient feature detector 208 that can be configured to identify a location for one or more transient features (e.g., spike noise) that may exist in each electrophysiological signal. For example, the transient feature is a spike that can correspond to a naturally occurring biological event (e.g., an arrhythmia, such a fibrillation) or the spike can be a pacing spike induced by a device. In some examples, the transient feature detector 208 is programmed to communicate transient location information for the transient feature in the given electrophysiological signal to the noise filtering system 202 for noise estimation. For example, the noise filtering system 202 is configured to employ the transient location information for the transient feature for the extraction of the signal segment of interest from the given electrophysiological signal. The transient location information can include timing or spatial location information for the transient feature.

In some examples, the transient feature detector 208 includes a transient feature removal function 210. The transient feature removal function 210 can be configured to remove the transient feature from the noise filtered signal data 206 based on the transient location information. The transient feature removal function 210 can be programmed to communicate the noise filtered signal data 206 without the transient feature to a low-pass filter 212. The low-pass filter 212 can be configured to attenuate or block frequencies higher than a predetermined cutoff frequency in the noise filtered signal data 206 to remove high-frequency signals (e.g., muscle artifacts and/or external interferences). The low-pass filter 212 can be configured to provide filtered input electrical signal data 214. Additional signal processing techniques can be employed on the filtered input electrical signal data 214. For example, electrogram reconstruction can be performed onto an epicardial or other envelope, such as by solving an inverse solution based on geometry data and the filtered input electrical signal data 214.

In some examples, the low-pass filter 212 can be implemented as an elliptic low-pass filter. By combining the noise filtering system 202 with the elliptic low-pass filter can reduce high frequency components in graphical maps of a heart surface that may be generated by a map generator, such as described herein, thereby leading to a more accurate representation of the electrical activity following a spike, which improves patient diagnosis.

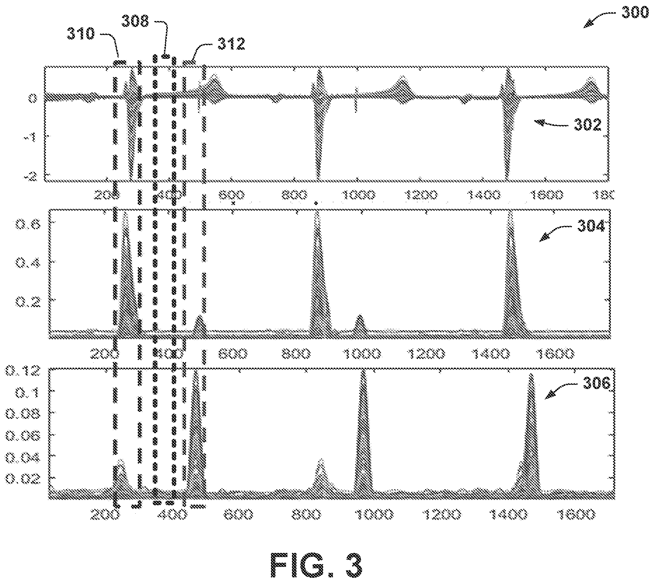

FIG. 3 depicts an example of electrophysiological signals and frequency-spectrum plots 300. A first plot 302 illustrates a composite plot of electrophysiological signals measured from a human's body (e.g., via sensors distributed across a surface of a patient's body). A second plot 304 illustrates a frequency-spectrum response of the electrophysiological signals in the first plot 302 emphasizing a QRS component of each of the electrophysiological signals in the first plot 302. A third plot 306 illustrates a frequency-spectrum response of the electrophysiological signals in the first plot 302 emphasizing a spike component within each of the electrophysiological signals. As described herein, a noise filtering system (e.g., the noise filtering system 100, as illustrated in FIG. 1 or the noise filtering system 202, as illustrated in FIG. 2), can be employed to apply a moving window function (e.g., a short window DFT (SWDFT)), such as from left to right in the first plot 302 to identify a signal segment of interest 308 of a given electrophysiological signal.

As illustrated in FIG. 3, the signal segment of interest 308 can be between a first remaining signal segment of interest 310 that includes the QRS component and a second remaining signal segment 312 that includes the spike component. The noise filtering system (e.g., system 100, 202) can employ the signal segment of interest 308 to estimate the noise in a signal segment of interest 308 and employ the estimated noise for filtering noise in the remaining signal segments 312 and 314. The noise filtering system can further remove the noise from the signal segment of interest 308 based on the estimated noise. The noise filtering system can combine the noise filtered segments 308, 310, and 312 to provide a noised filtered electrophysiological signal for further signal processing (e.g., removal of the spike component, low-pass filtering, and the like). In some examples, the noise filtering system can be configured to employ the estimated noise from the signal segment of interest 308 to filter noise in each remaining electrophysiological signal in the first plot 302 for further signal processing, such as described herein.

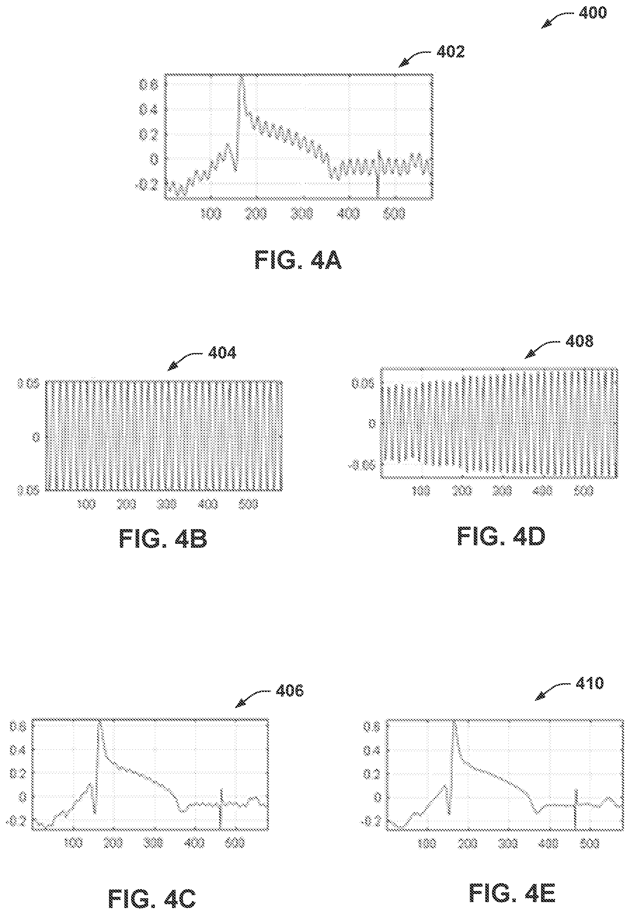

FIGS. 4A-4E depict examples of electrophysiological signal and noise filtering plots 400. In the example of FIGS. 4A-4E, a first plot 402 illustrates an unfiltered electrophysiological signal measured from a human's body (e.g., via a sensor), a second plot 404 illustrates an example of estimated system (e.g., global) noise for the electrophysiological signal 402. The third plot 406 illustrates an example of a system-noise filtered version of the electrophysiological signal in the first plot 402 based on removing the estimated system noise.

The plot 408 depicts an example of estimated noise for a signal segment of interest and the plot 410 illustrates an example of a noise-filtered version of the signal 402 responsive to removing the estimated noise 408 according to the approach described herein. For example, a noise filtering system (e.g., the noise filtering system 100, as illustrated in FIG. 1 or the noise filtering system 202, as illustrated in FIG. 2) can be configured to receive the electrophysiological signal of the first plot 402 and compute the estimated noise as illustrated in the plot 408 based on the segment of interest of the electrophysiological signal. The noise filtering system can employ the estimated noise to remove the noise in the signal segment of interest and remaining signal segments of the electrophysiological signal to provide a noise filtered electrophysiological signal, as illustrated in the plot 410.

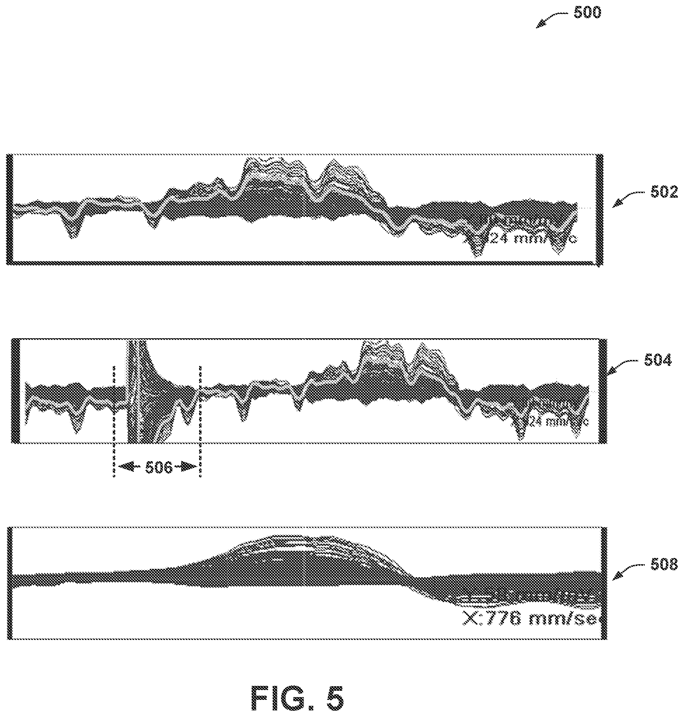

FIG. 5 depicts another example of electrophysiological signal plots 500. In some examples, a plurality of electrophysiological signals measured from a human's body can be provided to a GUI for rendering on a display. A first plot 502 illustrates a composite plot of the plurality of electrophysiological signals rendered by the GUI on the display without a spike component. A second plot 504 illustrates a composite plot of the plurality of electrophysiological signals rendered by the GUI on the display with the spike component. Because the spike component can distort the plurality of electrophysiological signals the spike component can be removed from the plurality in the plurality of electrophysiological signals.

For example, the GUI can generate GUI elements that a user can employ to interact with the plurality of electrophysiological signals. The user can further interact with the GUI elements to select or mark to define a time interval 506 corresponding to a spike component to exclude the spike component from further processing, such as inverse reconstruction. For example, a transient feature removal function (e.g., the transient feature removal function 210, as illustrated in FIG. 2) of a signal processing system can be configured to exclude the spike component from the electrophysiological signals in response to defining the time interval 506 for the spike component. A third plot 508 illustrates the electrophysiological signals without the spike component and filtered for noise by a noise filtering system (e.g., the system 100, 202), such as described herein.

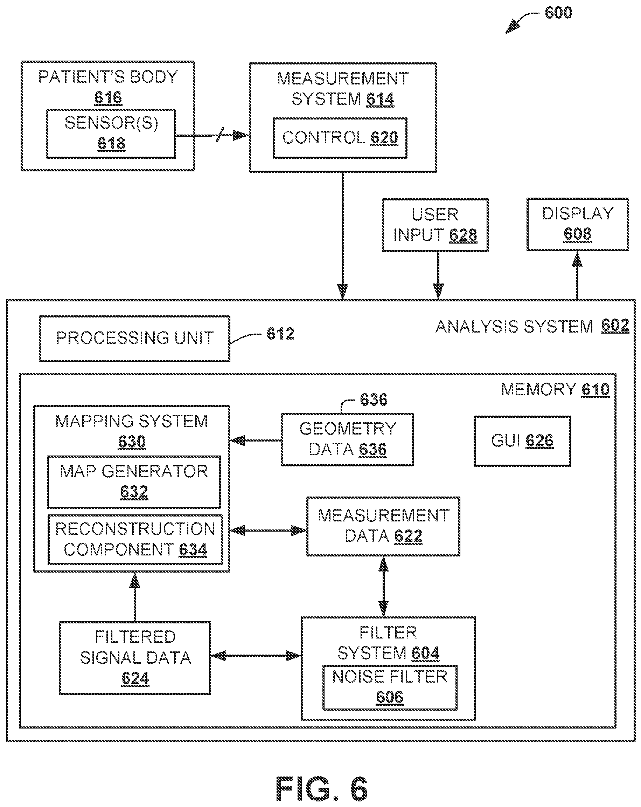

FIG. 6 depicts an example of an electrophysiological monitoring system 600 that can implement electrophysiological noise filtering as disclosed herein. The system 600 can include an analysis system 602 that employs a filter system 604, as disclosed herein (e.g., corresponding to the noising filtering system 100 of FIG. 1). In some examples, the filter system 604 can correspond to the signal processing system 200, as illustrated in FIG. 2. The filter system 604 can apply a noise filter 606 in real-time, such as during an electrophysiology study of a patient, or it can be implemented in relation to stored electrical measurement data previously acquired for a given patient. In some examples, the sensed electrical activity can be used to generate one or more graphical representations (e.g., graphical maps of electroanatomic activity) based on the sensed electrical activity, which can be provided to a display 608.

The analysis system 602 can be implemented as including a computer, such as a laptop computer, a desktop computer, a server, a tablet computer, a workstation, or the like. The analysis system 602 can include memory 610 for storing data and machine-readable instructions. The memory 610 can be implemented, for example, as a non-transitory computer storage medium, such as volatile memory (e.g., random access memory), non-volatile memory (e.g., a hard disk drive, a solid-state drive, flash memory, or the like) or a combination thereof. The instructions can be programmed to perform one or more methods, such as disclosed herein with respect to the example of FIG. 1.

The analysis system 602 can also include a processing unit 612 to access the memory 610 and execute the machine-readable instructions stored in the memory. The processing unit 612 could be implemented, for example, as one or more processor cores. In the present examples, although the components of the analysis system 602 are illustrated as being implemented on the same system, in other examples, the different components could be distributed across different systems and communicate, for example, over a network.

The system 600 can include a measurement system 614 to acquire electrophysiology information for a patient 616. In the example of FIG. 6, a sensor array 618 includes one or more electrodes that can be utilized for recording patient electrical activity. As one example, the sensor array 618 can correspond to an arrangement of body surface electrodes that are distributed over and around the patient's thorax for measuring electrical activity associated with the patient's heart (e.g., as part of an ECM procedure). In some examples, there can be about 200 or more sensors (e.g., about 252 sensors) in the sensor array 618, each sensor corresponding to a node that defines a respective channel. Examples of a non-invasive sensor array that can be used is shown and described in U.S. Patent No. 9,655,561, which was filed on December 22, 2011, or International patent application No. PCT/US2009/063803, which was filed 10 November 2009. This non-invasive sensor array corresponds to one example of a full complement of sensors that can include one or more sensing zones. As another example, the sensor array 618 can include an application-specific arrangement of electrodes corresponding to a single sensing zone or multiple discrete sensing zones. Additionally or alternatively, the sensor array 618 can include invasive sensors that can be inserted into the patient's body, such as via a catheter or other probe device.

The measurement system 614 receives sensed electrical signals from the corresponding sensor array 618. The measurement system 614 can include appropriate controls and signal processing circuitry (e.g., filters and safety circuitry) 620 for providing corresponding electrical measurement data 622 that describes electrical activity for each of a plurality of input channels detected by the sensors in the sensor array 618. In some examples, the electrical measurement data 622 corresponds to the input electrical signal data 102, as illustrated in FIG. 1.

The measurement data 622 can be stored in the memory 610 as analog or digital information. Appropriate time stamps and channel identifiers can be utilized for indexing the respective measurement data 622 to facilitate the evaluation and analysis thereof. As an example, each of the sensors in the sensor array 618 can simultaneously sense body surface electrical activity and provide corresponding measurement data 622 for one or more user-selected time intervals. Thus, the measurement data 622 can represent spatially and temporally consistent electrical information based on where the sensors in the array 618 are position on and/or in a body of the patient 616. The analysis system 602 is programmed to process the electrical measurement data 622 and to generate one or more outputs. The output can be stored in the memory 610 and provided to the display 608 or other type of output device. As disclosed herein, the type of output and information presented can vary depending on, for example, application requirements of the user.

As mentioned, the analysis system 602 is programmed to employ noise filter 606 to remove noise and/or transients from the measured electrical activity, which can results in improved accuracy in processing and analysis performed by the analysis system 602. The noise filter 606 can, for example, be implemented to perform any one or combination of the filter functions disclosed herein (see, e.g., FIGS. 1-2 and the corresponding description). The noise filter 606 thus can be applied to remove noise, transients, or other signal features from signal stored in the memory as the measurement data 622. The filter system 604 can be programmed to provide filtered signal data 624, including results from the noise filter 606, which are stored in the memory 610, such as in conjunction with the measurement data 622 and other parameter data. In some examples, the filtered signal data 624 corresponds to the noised filtered signal data 124, as illustrated in FIG. 1, or the noise filtered signal data 206, as illustrated in FIG. 2. In other examples, the filtered signal data 624 can correspond to the filtered input electrical signal data 214, as illustrated in FIG. 2.

In some examples, the filter system 604 can be programmed to interface with a graphical user interface (GUI) 626 stored as executable instructions in the memory 610. The GUI 626 thus can provide an interactive user interface, such as can be utilized to selectively define a time interval for processing electrophysiological signals in response to a user input 628. The GUI 626 can be programmed to provide data that can be rendered as interactive graphics on the display 608. For example, the GUI 626 can be programmed to generate GUI elements (e.g., check boxes, radio buttons, sliding scales, or the like) that a user can employ to select or mark to define or select a time interval corresponding to a filtering window for application to an input signal provided in the measurement data 622 for noise estimation as described herein.

The analysis system 602 can also generate an output to be presented graphically on the display 608 representing filtered or unfiltered waveforms for one or more signals. As disclosed herein, the waveforms can represent filtered or unfiltered graphical representations of respective input channels, similar to waveforms demonstrated in FIGS. 3-5. Alternatively, the output waveforms can represent filtered or unfiltered graphical representations of reconstructed waveforms, such as disclosed herein. As a further example, the analysis system 602 can include a mapping system 630 that can be programmed to generate an electroanatomical map based on the filtered signal data. The mapping system 630 can include a map generator 632 that can be programmed to generate map data representing a graphical (e.g., an electrical or electroanatomical map) based on the measurement data 622. The map generator 632 can be programmed to generate the map data to visualize a map via the display 608 spatially superimposed on a graphical representation of an anatomical structure (e.g., the heart).

In some examples, the mapping system 630 includes a reconstruction component 634 programmed to reconstruct heart electrical activity by combining the measurement data 622 with geometry data 636 through an inverse calculation. The inverse calculation employs a transformation matrix and reconstructs the electrical activity sensed by the sensor array 618 on the patient's body onto an anatomic envelope, such as an epicardial surface, an endocardial surface, or other envelope. Examples of inverse algorithms that can be implemented by the reconstruction component 634 are disclosed in U.S. Patent Nos. 7,983,743 and 6,772,004. The reconstruction component 634, for example, computes coefficients for a transfer matrix to determine heart electrical activity on a cardiac envelope based on the body surface electrical activity represented by the electrical measurement data 622. Since the reconstruction onto the envelope can be sensitive noise on the respective input channels, the filter system 604 helps to remove noise in channels that can distort signal morphology, which can improve patient diagnosis (e.g., for arrhythmia patients, such as CRT, PVC, VTs and PACs).

The map generator 632 can employ the reconstructed electrical data computed via the inverse method to produce a corresponding map of electrical activity. The map can represent an electrical activity of the patient's heart on the display 608, such as corresponding to a map of reconstructed electrograms (e.g., a potential map). Alternatively or additionally, an analysis system 602 can compute other electrical characteristics from the reconstructed electrograms, such as an activation map, a repolarization map, a propagation map, or other electrical characteristics that can be computed from the measurement data. The type of map can be set in response to the user input 628 via the GUI 626.

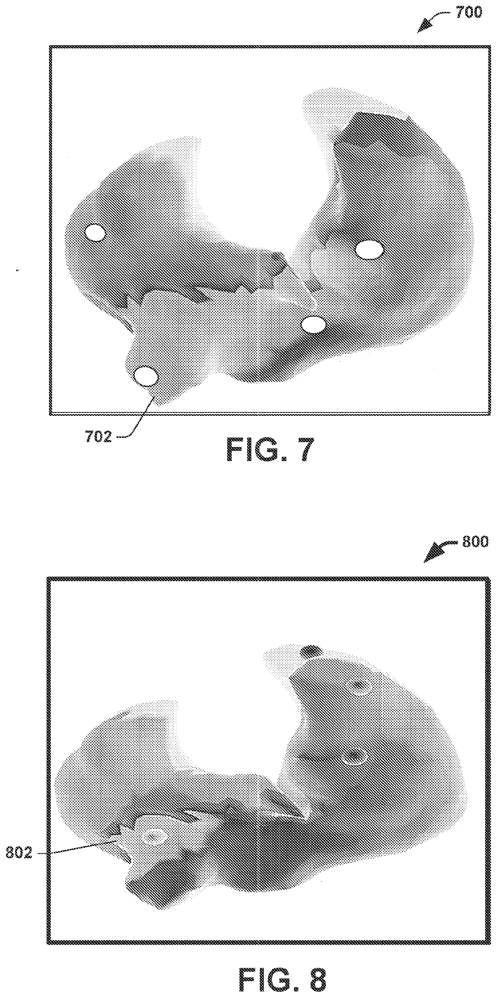

FIGS. 7-8 illustrate graphical maps 700 and 800 of electrical activity. Each graphical map can be generated by a map generator, such as the map generator 632, as illustrated in FIG. 6. FIG. 7 illustrates a graphical map 700 generated based on electrical signals measured from a body surface of a patient that has been filtered according to a different signal processing scheme as described herein. The electrical signals measured from the body surface of the patient for generating the graphical map 700 have been lined filtered using an infinite impulse response (IR) filter. As shown at 702, the graphical map 700 includes high-frequency components. FIG. 8 illustrates a graphical map 800 generated based on electrical signals measured from a body surface of a patient that has been filtered according to the processing scheme, as described herein, such as with respect to the example of FIG. 2. For example, the graphical map 800 can be generated based on the filtered input electrical signal data 214 that can be provided by the signal processing system 200. Because the signal processing system 200 employs the noise filtering system 202 with an elliptic low-pass filter as the low-pass filter 212 to filter the input electrical signal 204 such a combination reduces the high-frequency components in the graphical map 800, as illustrated at 802, thereby leading to a more accurate representation of the electrical activity following a spike, which improves patient diagnosis.

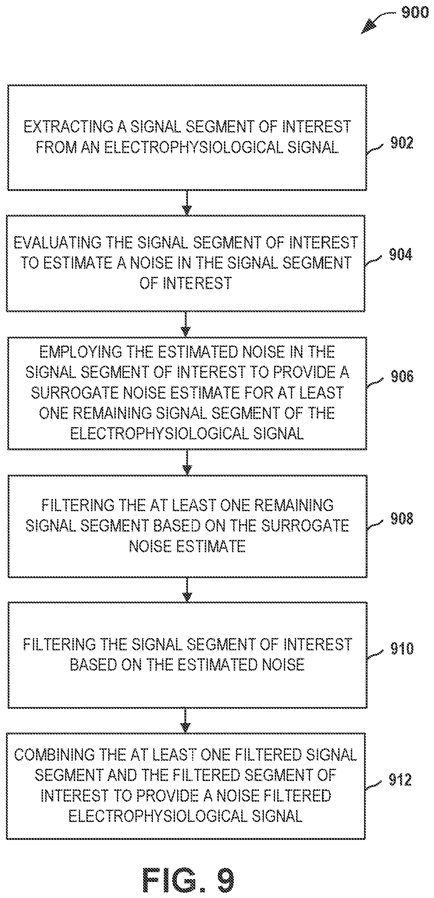

In view of the foregoing structural and functional features described above, an example method will be better appreciated with reference to FIG. 9. While, for purposes of simplicity of explanation, the example method of FIG. 9 is shown and described as executing serially, it is to be understood and appreciated that the example method is not limited by the illustrated order, as some actions could in other examples occur in different orders, multiple times and/or concurrently from that shown and described herein.

FIG. 9 illustrates an example of a method 900 for filtering noise from an electrophysiological signal. At least some portions of the method 900 can be implemented by hardware, software, or a combination of hardware and/or software, such as described herein. In some examples, the method can be implemented by a noise filtering system, such as the noise filtering system 100, as illustrated in FIG. 1 or the noise filtering system 202, as illustrated in FIG. 2. The method 900 can begin at 902 by extracting (e.g., via the signal segment extractor 104, as illustrated in FIG. 1) a signal segment of interest from an electrophysiological signal. At 904, the extracted signal segment of interest can be evaluated (e.g., by the signal segment noise calculator 110, as illustrated in FIG. 1) to estimate a noise in the signal segment of interest. At 906, the estimated noise in the signal segment of interest can be employed (e.g., via the signal segment filter 118, as illustrated in FIG. 1) to provide a surrogate noise estimate for at least one remaining signal segment of the electrophysiological signal. At 908, the at least one remaining signal segment of the electrophysiological signal can be filtered (e.g., via the signal segment filter 118, as illustrated in FIG. 1) to remove a noise in the at least one remaining signal segment to provide an at least one filtered remaining signal segment based on the surrogate noise estimate. At 910, the signal segment of interest of the electrophysiological signal can be filtered (e.g., via the signal segment filter 118, as illustrated in FIG. 1) to the remove the noise in the signal segment of interest to provide a filtered signal segment of interest based on the estimated noise. At 912, the at least one filtered remaining signal segment and the filtered signal segment of interest can be combined (e.g., via the signal segment filter 118, as illustrated in FIG. 1) to provide a noise filtered electrophysiological signal (e.g., as part of the noise filtered signal data 124, as illustrated in FIG. 1).

In view of the foregoing structural and functional description, those skilled in the art will appreciate that portions of the systems and method disclosed herein may be embodied as a method, data processing system, or computer program product such as a non-transitory computer readable medium. Accordingly, these portions of the approach disclosed herein may take the form of an entirely hardware embodiment, an entirely software embodiment (e.g., in a non-transitory machine readable medium), or an embodiment combining software and hardware. Furthermore, portions of the systems and method disclosed herein may be a computer program product on a computer-usable storage medium having computer readable program code on the medium. Any suitable computer-readable medium may be utilized including, but not limited to, static and dynamic storage devices, hard disks, optical storage devices, and magnetic storage devices.

Certain embodiments have also been described herein with reference to block illustrations of methods, systems, and computer program products. It will be understood that blocks of the illustrations, and combinations of blocks in the illustrations, can be implemented by computer-executable instructions. These computer-executable instructions may be provided to one or more processor of a general purpose computer, special purpose computer, or other programmable data processing apparatus (or a combination of devices and circuits) to produce a machine, such that the instructions, which execute via the processor, implement the functions specified in the block or blocks.

These computer-executable instructions may also be stored in computer-readable memory that can direct a computer or other programmable data processing apparatus to function in a particular manner, such that the instructions stored in the computer-readable memory result in an article of manufacture including instructions which implement the function specified in the flowchart block or blocks. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions described herein.

What have been described above are examples. It is, of course, not possible to describe every conceivable combination of structures, components, or methods, but one of ordinary skill in the art will recognize that many further combinations and permutations are possible. Accordingly, the invention is defined by the appended claims. Where the disclosure or claims recite "a," "an," "a first," or "another" element, or the equivalent thereof, it should be interpreted to include one or more than one such element, neither requiring nor excluding two or more such elements. As used herein, the term "includes" means includes but not limited to, and the term "including" means including but not limited to. The term "based on" means based at least in part on.

## Claims

1. One or more non-transitory computer-readable media having data and machine readable instructions executable by a processor, the data comprising electroanatomical data characterizing an electrophysiological signal measured from a patient, the machine readable instructions comprising:

   a signal segment extractor programmed to extract a signal segment of interest from the electrophysiological signal;
   a signal segment noise calculator programmed to evaluate the extracted signal segment of interest to estimate a noise in the signal segment of interest; and
   a signal segment filter programmed to determine a surrogate noise estimate for at least one remaining signal segment of the electrophysiological signal based on the estimated noise in the signal segment of interest, and filter the at least one remaining signal segment to remove noise therein based on the surrogate noise estimate, the at least one remaining signal segment being different from the extracted signal segment,

   wherein the signal segment extractor is programmed to apply a moving window function to sample a portion of the electrophysiological signal and evaluate a signal morphology of the sampled portion of the electrophysiological signal to determine whether the portion of the electrophysiological signal is to be identified as the signal segment of interest.

2. The one or more non-transitory computer-readable media of claim 1, wherein the moving window function includes a Hamming window.

3. The one or more non-transitory computer-readable media of claim 2, wherein the signal segment noise calculator comprises a transform function programmed to convert the sampled signal segment of interest to corresponding frequency domain data having discrete frequency bins for signals in the signal segment of interest.

4. The one or more non-transitory computer-readable media of claim 3, wherein the transform function is programmed to apply discrete Fourier transform (DFT) to the sampled signal segment of interest to convert the sampled signal segment of interest to the corresponding frequency domain data.

5. The one or more non-transitory computer-readable media of claim 4, wherein the signal segment noise calculator is programmed to compute a set of DFT coefficients that include a phase and an amplitude for each signal in the signal segment of interest to convert the sampled signal segment of interest to the corresponding frequency domain data.

6. The one or more non-transitory computer-readable media of claim 5, wherein the signal segment noise calculator is programmed to:

evaluate the corresponding frequency domain data to identify a frequency of a noise signal among respective signals in the signal segment of interest;
select a DFT coefficient of the set of DFT coefficients for the noise signal based on the identified frequency; and
estimate the noise in the signal segment of interest based on the selected DFT coefficient.

7. The one or more non-transitory computer-readable media of claim 6, wherein the signal segment filter comprises:

an extended window function programmed to sample the at least one remaining signal segment of the electrophysiological signal; and
a noise estimation function programmed to extrapolate or interpolate the estimated noise in the signal segment of interest to the at least one remaining signal segment to provide the surrogate noise estimate for the at least one remaining signal segment based on the selected DFT coefficient for the noise signal.

8. The one or more non-transitory computer-readable media of claim 7, wherein the signal segment noise calculator is programmed to scale the selected DFT coefficient to scale the estimated noise in the signal segment of interest noise estimation in the at least one remaining signal segment.

9. The one or more non-transitory computer-readable media of claim 7 or claim 8, wherein the signal segment filter comprises a segment filter function programmed to subtract the noise in the at least one remaining signal segment from the surrogate noise estimate for the at least one remaining signal segment to filter the electrophysiological signal for the noise.

10. The one or more non-transitory computer-readable media of claim 9, wherein the noise in the electrophysiological signal is line noise having a frequency of one of 50 Hertz (Hz) and 60 Hz, and the signal segment of interest does not comprise one of a spike and a QRS complex.

11. The one or more non-transitory computer-readable media of claim 10,

wherein the electroanatomical data comprises a plurality of electrophysiological signals measured from the patient via a set of sensors, and the electrophysiological signal correspond to a given electrophysiological signal, and
wherein the noise estimation function is programmed to provide a surrogate noise estimate for remaining electrophysiological signals of the plurality of electrophysiological signals based on the estimated noise in the signal segment of interest of the given electrophysiological signal, and the segment filter function is programmed subtract a noise in the remaining electrophysiological signals from the surrogate noise estimate for the remaining electrophysiological signals to filter the remaining electrophysiological signals for the noise.

12. The one or more non-transitory computer-readable media of claim 11, wherein the machine readable instructions comprise a plurality of noise filtering systems respectively comprising the signal segment extractor, the signal segment noise calculator, and the signal segment filter, and the set of sensors are arranged in a plurality of spatial zones, and wherein a respective noise filtering system of the plurality of noise filtering systems is adapted to be employed for each spatial zone, and configured to:

estimate a noise in a signal segment of interest of a respective electrophysiological signal measured by a sensor of a respective spatial zone of the plurality of spatial zones, the respective electrophysiological signal corresponding to the given electrophysiological signal;
compute a surrogate noise estimate for electrophysiological signals measured by remaining sensors of the respective spatial zone based on the estimated noise in the signal segment of interest of the respective electrophysiological signal; and
subtract a noise in the electrophysiological signals measured by the remaining sensors of the respective spatial zone from the surrogate noise estimate for the electrophysiological signals to filter the electrophysiological signals for the noise.

13. The one or more non-transitory computer-readable media of claim 12, wherein the signal segment of interest is a first signal segment of interest, the surrogate noise is a first surrogate noise, and the data comprises electrical signal data characterizing a signal generated by a therapeutic device or a navigation system, wherein the signal segment extractor is programmed to extract a second signal segment of interest from the signal, the signal segment noise calculator is programmed to evaluate the second signal segment of interest to estimate the noise in the second signal segment of interest, and the signal segment filter is programmed to determine a second surrogate noise estimate for at least one remaining signal segment of the signal and filter the at least one remaining signal segment of the signal to remove noise therein based on the second surrogate noise estimate.

14. The one or more non-transitory computer-readable media of claim 12 or 13, wherein the data comprises electrical signal data characterizing measured electrical signals applied to a body of the patient, wherein the signal segment extractor is programmed to:

   evaluate the measured electrical signals to determine a common noise in the measured electrical signals; and
   extract the signal segment of interest based on the determined common noise in the measured electrical signals.

15. The one or more non-transitory computer-readable media of any of claims 12-14, the machine-readable instructions further comprising instructions for causing a mapping system to:

   generate a first graphical map of electroanatomic activity based on electrophysiological signals provided by respective sensors of one or more first spatial zones of the plurality of spatial zones;
   generate a second graphical map of electroanatomic activity based on electrophysiological signals provided by respective sensors of one or more second spatial zones of the plurality of spatial zones; and
   evaluate the first and second graphical maps of the electroanatomic activity to determine a quality of noise filtering for each of the first and second spatial zones of the plurality of spatial zones,

   and/or
   wherein the respective noise filtering system further comprises a machine learning algorithm programmed to refine a noise filtering of the respective noise filtering system based on historical noise filtering data.

**Patentansprüche**

1. Ein oder mehrere nichtflüchtige computerlesbare Medien mit Daten und maschinenlesbaren Anweisungen, die durch einen Prozessor ausführbar sind, wobei die Daten elektroanatomische Daten umfassen, die ein gemessenes elektrophysiologisches Signal von einem Patienten charakterisieren, wobei die maschinenlesbaren Anweisungen umfassen:

   einen Signalsegmentextrahierer, der dazu programmiert ist, ein Signalsegment von Interesse aus dem elektrophysiologischen Signal zu extrahieren;
   einen Signalsegment-Rauschrechner, der dazu programmiert ist, das extrahierte Signalsegment von Interesse auszuwerten, um ein Rauschen im Signalsegment von Interesse zu schätzen; und
   ein Signalsegmentfilter, das dazu programmiert ist, eine Ersatzrauschschätzung für mindestens ein verbleibendes Signalsegment des elektrophysiologischen Signals basierend auf dem geschätzten Rauschen im Signalsegment von Interesse zu bestimmen und das mindestens eine verbleibende Signalsegment zu filtern, um Rauschen darin zu entfernen, basierend auf der Ersatzrauschschätzung, wobei das mindestens eine verbleibende Signalsegment von dem extrahierten Signalsegment verschieden ist, wobei der Signalsegmentextrahierer dazu programmiert ist, eine Gleitfensterfunktion anzuwenden, um einen Teil des elektrophysiologischen Signals abzutasten und eine Signalmorphologie des abgetasteten Teils des elektrophysiologischen Signals auszuwerten, um zu bestimmen, ob der Teil des elektrophysiologischen Signals als das Signalsegment von Interesse zu identifizieren ist.

2. Ein oder mehrere nichtflüchtige computerlesbare Medien nach Anspruch 1, wobei die Gleitfensterfunktion ein Hamming-Fenster enthält.

3. Ein oder mehrere nichtflüchtige computerlesbare Medien nach Anspruch 2, wobei der Signalsegment-Rauschrechner eine Transformationsfunktion umfasst, die dazu programmiert ist, das abgetastete Signalsegment von Interesse in entsprechende Frequenzdomänendaten mit diskreten Frequenzbins für Signale im Signalsegment von Interesse umzuwandeln.

4. Ein oder mehrere nichtflüchtige computerlesbare Medien nach Anspruch 3, wobei die Transformationsfunktion dazu programmiert ist, eine diskrete FourierTransformation (DFT) auf das abgetastete Signalsegment von Interesse anzuwenden, um das abgetastete Signalsegment von Interesse in die entsprechenden Frequenzdomänendaten umzuwandeln.

5. Ein oder mehrere nichtflüchtige computerlesbare Medien nach Anspruch 4, wobei der Signalsegment-Rauschrechner dazu programmiert ist, einen Satz von DFT-Koeffizienten zu berechnen, die eine Phase und eine Amplitude für jedes Signal im Signalsegment von Interesse enthalten, um das abgetastete Signalsegment von Interesse in die entsprechenden Frequenzdomänendaten umzuwandeln.

6. Ein oder mehrere nichtflüchtige computerlesbare Medien nach Anspruch 5, wobei der Signalsegment-Rauschrechner programmiert ist zum:

Auswerten der entsprechenden Frequenzdomänendaten, um eine Frequenz eines Rauschsignals unter den jeweiligen Signalen im Signalsegment von Interesse zu identifizieren; Auswählen eines DFT-Koeffizienten des Satzes von DFT-Koeffizienten für das Rauschsignal basierend auf der identifizierten Frequenz; und Schätzen des Rauschens im Signalsegment von Interesse basierend auf dem ausgewählten DFT-Koeffizienten.

7. Ein oder mehrere nichtflüchtige computerlesbare Medien nach Anspruch 6, wobei das Signalsegmentfilter umfasst:

eine erweiterte Fensterfunktion, die dazu programmiert ist, das mindestens eine verbleibende Signalsegment des elektrophysiologischen Signals abzutasten; und eine Rauschschätzfunktion, die dazu programmiert ist, das geschätzte Rauschen im Signalsegment von Interesse auf das mindestens eine verbleibende Signalsegment zu extrapolieren oder zu interpolieren, um die Ersatzrauschschätzung für das mindestens eine verbleibende Signalsegment bereitzustellen, basierend auf dem ausgewählten DFT-Koeffizienten für das Rauschsignal.

8. Ein oder mehrere nichtflüchtige computerlesbare Medien nach Anspruch 7, wobei der Signalsegment-Rauschrechner dazu programmiert ist, den ausgewählten DFT-Koeffizienten zu skalieren, um das geschätzte Rauschen in der Rauschschätzung des Signalsegments von Interesse in dem mindestens einen verbleibenden Signalsegment zu skalieren.

9. Ein oder mehrere nichtflüchtige computerlesbare Medien nach Anspruch 7 oder 8, wobei das Signalsegmentfilter eine Segmentfilterfunktion umfasst, die dazu programmiert ist, das Rauschen in dem mindestens einen verbleibenden Signalsegment von der Ersatzrauschschätzung für das mindestens eine verbleibende Signalsegment zu subtrahieren, um das elektrophysiologische Signal auf das Rauschen hin zu filtern.

10. Ein oder mehrere nichtflüchtige computerlesbare Medien nach Anspruch 9, wobei das Rauschen im elektrophysiologischen Signal ein Netzbrummen mit einer Frequenz von 50 Hertz (Hz) und 60 Hz ist und das Signalsegment von Interesse keinen Spike oder QRS-Komplex umfasst.

11. Ein oder mehrere nichtflüchtige computerlesbare Medien nach Anspruch 10,

wobei die elektroanatomischen Daten eine Vielzahl von elektrophysiologischen Signalen umfassen, die vom Patienten über einen Satz von Sensoren gemessen werden, und das elektrophysiologische Signal einem gegebenen elektrophysiologischen Signal entspricht, und wobei die Rauschschätzfunktion dazu programmiert ist, eine Ersatzrauschschätzung für verbleibende elektrophysiologische Signale der Vielzahl von elektrophysiologischen Signalen basierend auf dem geschätzten Rauschen im Signalsegment von Interesse des gegebenen elektrophysiologischen Signals bereitzustellen, und die Segmentfilterfunktion dazu programmiert ist, ein Rauschen in den verbleibenden elektrophysiologischen Signalen von der Ersatzrauschschätzung für die verbleibenden elektrophysiologischen Signale zu subtrahieren, um die verbleibenden elektrophysiologischen Signale auf das Rauschen hin zu filtern.

12. Ein oder mehrere nichtflüchtige computerlesbare Medien nach Anspruch 11, wobei die maschinenlesbaren Anweisungen eine Vielzahl von Rauschfilterungssystemen umfassen, die jeweils den Signalsegmentextrahierer, den Signalsegment-Rauschrechner und das Signalsegmentfilter umfassen und der Satz von Sensoren in einer Vielzahl von räumlichen Zonen angeordnet ist, und wobei ein jeweiliges Rauschfilterungssystem aus der Vielzahl der Rauschfilterungssysteme dazu geeignet ist, für jede räumliche Zone eingesetzt zu werden, und ausgelegt ist zum:

Schätzen eines Rauschens in einem Signalsegment von Interesse eines jeweiligen elektrophysiologischen Signals, das durch einen Sensor einer jeweiligen räumlichen Zone der Vielzahl von räumlichen Zonen gemessen wird, wobei das jeweilige elektrophysiologische Signal dem gegebenen elektrophysiologischen Signal entspricht; Berechnen einer Ersatzrauschschätzung für elektrophysiologische Signale, die durch verbleibende Sensoren der jeweiligen räumlichen Zone gemessen werden, basierend auf dem geschätzten Rauschen im Signalsegment von Interesse des jeweiligen elektrophysiologischen Signals; und

Subtrahieren eines Rauschens in den durch die verbleibenden Sensoren der jeweiligen räumlichen Zone gemessenen elektrophysiologischen Signalen von der Ersatzrauschschätzung für die elektrophysiologischen Signale, um die elektrophysiologischen Signale auf das Rauschen hin zu filtern.

13. Ein oder mehrere nichtflüchtige computerlesbare Medien nach Anspruch 12, wobei das Signalsegment von Interesse ein erstes Signalsegment von Interesse ist, das Ersatzrauschen ein erstes Ersatzrauschen ist und die Daten elektrische Signaldaten umfassen, die ein durch eine Therapievorrichtung oder ein Navigationssystem erzeugtes Signal charakterisiert, wobei der Signalsegmentextrahierer dazu programmiert ist, ein zweites Signalsegment von Interesse aus dem Signal zu extrahieren, der Signalsegment-Rauschrechner dazu programmiert ist, das zweite Signalsegment von Interesse auszuwerten, um das Rauschen im zweiten Signalsegment von Interesse zu schätzen, und das Signalsegmentfilter dazu programmiert ist, eine zweite Ersatzrauschschätzung für mindestens ein verbleibendes Signalsegment des Signals zu bestimmen und das mindestens eine verbleibende Signalsegment des Signals zu filtern, um Rauschen darin zu entfernen, basierend auf der zweiten Ersatzrauschschätzung.

14. Ein oder mehrere nichtflüchtige computerlesbare Medien nach Anspruch 12 oder 13, wobei die Daten elektrische Signaldaten umfassen, die gemessene elektrische Signale charakterisieren, die an einen Körper des Patienten angelegt werden, wobei der Signalsegmentextrahierer programmiert ist zum:

Auswerten der gemessenen elektrischen Signale, um ein gemeinsames Rauschen in den gemessenen elektrischen Signalen zu bestimmen; und
Extrahieren des Signalsegments von Interesse basierend auf dem bestimmten gemeinsamen Rauschen in den gemessenen elektrischen Signalen.

15. Ein oder mehrere nichtflüchtige computerlesbare Medien nach einem der Ansprüche 12-14, wobei die maschinenlesbaren Anweisungen ferner Anweisungen umfassen, um ein Kartierungssystem zu veranlassen zum:

Erzeugen einer ersten grafischen Karte der elektroanatomischen Aktivität basierend auf elektrophysiologischen Signalen, die durch jeweilige Sensoren einer oder mehrerer erster räumlicher Zonen der Vielzahl räumlicher Zonen bereitgestellt werden;

Erzeugen einer zweiten grafischen Karte der elektroanatomischen Aktivität basierend auf elektrophysiologischen Signalen, die durch jeweilige Sensoren einer oder mehrerer zweiter räumlicher Zonen der Vielzahl räumlicher Zonen bereitgestellt werden; und
Auswerten der ersten und der zweiten graphischen Karte der elektroanatomischen Aktivität, um eine Qualität der Rauschfilterung für jede der ersten und zweiten räumlichen Zonen der Vielzahl von räumlichen Zonen zu bestimmen, und/oder
wobei das jeweilige Rauschfilterungssystem ferner einen Maschinenlernalgorithmus umfasst, der dazu programmiert ist, eine Rauschfilterung des jeweiligen Rauschfilterungssystems basierend auf historischen Rauschfilterungsdaten zu verfeinern.

## Revendications

1. Un ou plusieurs supports non transitoires lisibles par ordinateur contenant des données et des instructions lisibles par machine exécutables par un processeur, les données comprenant des données électroanatomiques caractérisant un signal électrophysiologique mesuré sur un patient, les instructions lisibles par machine comprenant :

un extracteur de segment de signal programmé pour extraire un segment de signal d'intérêt du signal électrophysiologique ;
un calculateur de bruit de segment de signal programmé pour évaluer le segment de signal d'intérêt extrait afin d'estimer un bruit dans le segment de signal d'intérêt ; et
un filtre de segment de signal programmé pour déterminer une estimation de bruit de substitution pour au moins un segment de signal restant du signal électrophysiologique en fonction du bruit estimé dans le segment de signal d'intérêt, et filtrer l'au moins un segment de signal restant pour éliminer le bruit dans celui-ci en fonction de l'estimation de bruit de substitution, l'au moins un segment de signal restant étant différent du segment de signal extrait,
dans lequel l'extracteur de segment de signal est programmé pour appliquer une fonction de fenêtre mobile pour échantillonner une partie du signal électrophysiologique et évaluer une morphologie de signal de la partie échantillonnée du signal électrophysiologique afin de déterminer si la partie du signal électrophysiologique doit être identifiée en tant que segment de signal d'intérêt.

2. Un ou plusieurs supports non transitoires lisibles par ordinateur selon la revendication 1, dans lesquels la fonction de fenêtre mobile comporte une fenêtre de Hamming.

3. Un ou plusieurs supports non transitoires lisibles par ordinateur selon la revendication 2, dans lesquels le calculateur de bruit de segment de signal comprend une fonction de transformée programmée pour convertir le segment de signal échantillonné d'intérêt en données de domaine fréquentiel correspondantes ayant des intervalles de fréquence discrets pour des signaux dans le segment de signal d'intérêt.

4. Un ou plusieurs supports non transitoires lisibles par ordinateur selon la revendication 3, dans lesquels la fonction de transformée est programmée pour appliquer une transformée de Fourier discrète (TFD) au segment de signal échantillonné d'intérêt pour convertir le segment de signal échantillonné d'intérêt en données de domaine fréquentiel correspondantes.

5. Un ou plusieurs supports non transitoires lisibles par ordinateur selon la revendication 4, dans lesquels le calculateur de bruit de segment de signal est programmé pour calculer un ensemble de coefficients de TFD qui comportent une phase et une amplitude pour chaque signal dans le segment de signal d'intérêt pour convertir le segment de signal échantillonné d'intérêt en données de domaine fréquentiel correspondantes.

6. Un ou plusieurs supports non transitoires lisibles par ordinateur selon la revendication 5, dans lesquels le calculateur de bruit de segment de signal est programmé pour :

   évaluer les données de domaine fréquentiel correspondantes pour identifier une fréquence d'un signal de bruit parmi des signaux respectifs dans le segment de signal d'intérêt ;
   sélectionner un coefficient de TFD de l'ensemble de coefficients de TFD pour le signal de bruit en fonction de la fréquence identifiée ; et
   estimer le bruit dans le segment de signal d'intérêt en fonction du coefficient de TFD sélectionné.

7. Un ou plusieurs supports non transitoires lisibles par ordinateur selon la revendication 6, dans lesquels le filtre de segment de signal comprend :

   une fonction de fenêtre étendue programmée pour échantillonner l'au moins un segment de signal restant du signal électrophysiologique ; et
   une fonction d'estimation de bruit programmée pour extrapoler ou interpoler le bruit estimé dans le segment de signal d'intérêt vers l'au moins un segment de signal restant afin de fournir l'estimation de bruit de substitution pour l'au moins un segment de signal restant en fonction du coefficient de TFD sélectionné pour le signal de bruit.

8. Un ou plusieurs supports non transitoires lisibles par ordinateur selon la revendication 7, dans lesquels le calculateur de bruit de segment de signal est programmé pour mettre à l'échelle le coefficient de TFD sélectionné pour mettre à l'échelle le bruit estimé dans l'estimation de bruit du segment de signal d'intérêt dans l'au moins un segment de signal restant.

9. Un ou plusieurs supports non transitoires lisibles par ordinateur selon la revendication 7 ou la revendication 8, dans lesquels le filtre de segment de signal comprend une fonction de filtre de segment programmée pour soustraire le bruit dans l'au moins un segment de signal restant de l'estimation de bruit de substitution pour l'au moins un segment de signal restant afin de filtrer le bruit du signal électrophysiologique.

10. Un ou plusieurs supports non transitoires lisibles par ordinateur selon la revendication 9, dans lesquels le bruit dans le signal électrophysiologique est un bruit de ligne ayant une fréquence de 50 hertz (Hz) ou 60 Hz, et le segment de signal d'intérêt ne comprend pas de pic ou de complexe QRS.

11. Un ou plusieurs supports non transitoires lisibles par ordinateur selon la revendication 10,

   dans lesquels les données électroanatomiques comprennent une pluralité de signaux électrophysiologiques mesurés à partir du patient par le biais d'un ensemble de capteurs, et le signal électrophysiologique correspond à un signal électrophysiologique donné, et
   dans lequel la fonction d'estimation de bruit est programmée pour fournir une estimation de bruit de substitution pour les signaux électrophysiologiques restants de la pluralité de signaux électrophysiologiques en fonction du bruit estimé dans le segment de signal d'intérêt du signal électrophysiologique donné, et la fonction de filtre de segment est programmée pour soustraire un bruit dans les signaux électrophysiologiques restants de l'estimation de bruit de substitution pour les signaux électrophysiologiques restants afin de filtrer le bruit des signaux électrophysiologiques restants.

12. Un ou plusieurs supports non transitoires lisibles par ordinateur selon la revendication 11, dans lesquels les instructions lisibles par machine comprennent une pluralité de systèmes de filtrage de bruit comprenant respectivement l'extracteur de segment de signal, le calculateur de bruit de segment de signal et le filtre de segment de signal, et l'ensemble de capteurs est agencé dans une pluralité de zones spatiales, et

dans lequel un système de filtrage de bruit respectif de la pluralité de systèmes de filtrage de bruit est conçu pour être utilisé pour chaque zone spatiale, et configuré pour :

estimer un bruit dans un segment de signal d'intérêt d'un signal électrophysiologique respectif mesuré par un capteur d'une zone spatiale respective de la pluralité de zones spatiales, le signal électrophysiologique respectif correspondant au signal électrophysiologique donné ;

calculer une estimation du bruit de substitution pour les signaux électrophysiologiques mesurés par les capteurs restants de la zone spatiale respective en fonction du bruit estimé dans le segment de signal d'intérêt du signal électrophysiologique respectif ; et

soustraire un bruit dans les signaux électrophysiologiques mesurés par les capteurs restants de la zone spatiale respective de l'estimation de bruit de substitution pour les signaux électrophysiologiques afin de filtrer le bruit des signaux électrophysiologiques.

13. Un ou plusieurs supports non transitoires lisibles par ordinateur selon la revendication 12, dans lesquels le segment de signal d'intérêt est un premier segment de signal d'intérêt, le bruit de substitution est un premier bruit de substitution, et les données comprennent des données de signal électrique caractérisant un signal généré par un dispositif thérapeutique ou un système de navigation, dans lesquels l'extracteur de segment de signal est programmé pour extraire un second segment de signal d'intérêt du signal, le calculateur de bruit de segment de signal est programmé pour évaluer le second segment de signal d'intérêt afin d'estimer le bruit dans le second segment de signal d'intérêt, et le filtre de segment de signal est programmé pour déterminer une seconde estimation de bruit de substitution pour au moins un segment de signal restant du signal et filtrer l'au moins un segment de signal restant du signal afin d'éliminer le bruit dans celui-ci en fonction de la seconde estimation de bruit de substitution.

14. Un ou plusieurs supports non transitoires lisibles par ordinateur selon la revendication 12 ou 13, dans lesquels les données comprennent des données de signal électrique caractérisant des signaux électriques mesurés appliqués au corps du patient, dans lequel l'extracteur de segment de signal est programmé pour :

évaluer les signaux électriques mesurés pour déterminer un bruit commun dans les signaux électriques mesurés ; et

extraire le segment de signal d'intérêt en fonction du bruit commun déterminé dans les signaux électriques mesurés.

15. Un ou plusieurs supports non transitoires lisibles par ordinateur selon l'une quelconque des revendications 12 à 14, les instructions lisibles par machine comprenant en outre des instructions pour amener un système de mappage à :

générer une première carte graphique de l'activité électroanatomique en fonction de signaux électrophysiologiques fournis par des capteurs respectifs d'une ou de plusieurs premières zones spatiales de la pluralité de zones spatiales ;

générer une seconde carte graphique de l'activité électroanatomique en fonction de signaux électrophysiologiques fournis par des capteurs respectifs d'une ou de plusieurs secondes zones spatiales de la pluralité de zones spatiales ; et

évaluer les première et seconde cartes graphiques de l'activité électroanatomique pour déterminer une qualité de filtrage de bruit pour chacune des première et seconde zones spatiales de la pluralité de zones spatiales,

et/ou

dans lequel le système de filtrage de bruit respectif comprend en outre un algorithme d'apprentissage automatique programmé pour affiner un filtrage de bruit du système de filtrage de bruit respectif en fonction de données historiques de filtrage de bruit.

FIG. 1

200

```
┌─────────────────┐
│     INPUT       │
│   ELECTRICAL    │
│ SIGNAL DATA 204 │
└────────┬────────┘
         │
         ▼
┌─────────────────┐
│ NOISE FILTERING │◄─────────────┐
│  SYSTEM 202     │              │
└────────┬────────┘              │
         │                       │
         ▼              ┌────────┴──────────┐
┌─────────────────┐     │    TRANSIENT      │
│ NOISE FILTERED  │     │    FEATURE        │
│ SIGNAL DATA 206 ├──►  │  DETECTOR 208     │
└─────────────────┘     │ ┌───────────────┐ │
                        │ │  TRANSIENT    │ │
                        │ │  FEATURE      │ │
                        │ │  REMOVAL      │ │
                        │ │ FUNCTION 210  │ │
                        │ └───────────────┘ │
                        └────────┬──────────┘
```

TRANSIENT
FEATURE
DETECTOR 208

TRANSIENT
FEATURE
REMOVAL
FUNCTION 210

LOW PASS
FILTER 212

FILTERED INPUT
ELECTRICAL SIGNAL
DATA 214

# FIG. 2

FIG. 3

FIG. 4A

FIG. 4B

FIG. 4D

FIG. 4C

FIG. 4E

**FIG. 5**

FIG. 6

FIG. 7

FIG. 8

900

EXTRACTING A SIGNAL SEGMENT OF INTEREST FROM AN ELECTROPHYSIOLOGICAL SIGNAL — 902

EVALUATING THE SIGNAL SEGMENT OF INTEREST TO ESTIMATE A NOISE IN THE SIGNAL SEGMENT OF INTEREST — 904

EMPLOYING THE ESTIMATED NOISE IN THE SIGNAL SEGMENT OF INTEREST TO PROVIDE A SURROGATE NOISE ESTIMATE FOR AT LEAST ONE REMAINING SIGNAL SEGMENT OF THE ELECTROPHYSIOLOGICAL SIGNAL — 906

FILTERING THE AT LEAST ONE REMAINING SIGNAL SEGMENT BASED ON THE SURROGATE NOISE ESTIMATE — 908

FILTERING THE SIGNAL SEGMENT OF INTEREST BASED ON THE ESTIMATED NOISE — 910

COMBINING THE AT LEAST ONE FILTERED SIGNAL SEGMENT AND THE FILTERED SEGMENT OF INTEREST TO PROVIDE A NOISE FILTERED ELECTROPHYSIOLOGICAL SIGNAL — 912

**FIG. 9**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019183425 A1
- US 2014278171 A1
- US 9549683 B
- US 20091063803 W
- US 9655561 B
- US 2009063803 W
- US 7983743 B
- US 6772004 B

**Non-patent literature cited in the description**

- **GLASER et al.** *BMC Neuroscience*, 2013, vol. 14 (1), 138